# EUROPEAN PATENT APPLICATION

(11) **EP 2 243 448 A1**
(43) Date of publication of application: **27.10.2010**
(21) Application number: 09709533.5
(22) Date of filing: 12.02.2009
(51) Int. Cl.: A61F 5/44, A61F 5/451, A61F 5/457

(54) **AN AUTOMATED URINE PROCESSING APPARATUS**

(30) Priority: 14.02.2008 JP 2008033653
(71) Applicant: Uni-Charm Corporation, Ehime 799-0111 (JP); Hitachi, Ltd., Chiyoda-ku Tokyo 100-8280 (JP)
(72) Inventor: WADA, Ichiro, Kanonji-shi Kagawa 769-1602 (JP); SUZUKI, Miou, Kanonji-shi Kagawa 769-1602 (JP); MIYAGAWA, Ryosuke, Tokyo 101-8608 (JP); TANAKA, Tetsuya, Tokyo 101-8608 (JP); ISHITSUKA, Yoshikazu, Tokyo 101-8608 (JP)
(74) Representative: Fitchett, Stuart Paul
(86) International application number: PCT/JP2009/052292
(87) International publication number: WO 2009/101969

(57) **Abstract**

An automatic urine disposal apparatus adapted to detect the presence of feces in a wide range.

A detector unit in an automatic urine disposal apparatus has a urination detector 102b and a defecation detector 102c. The urination detector 102b comprises a pair of first electrodes 218a, 218b and the defecation detector 102c comprises a pair of second electrodes 143a, 143b. The pair of first electrodes 218a, 218b and the pair of second electrodes 143a, 143b respectively have regions spaced one from another and extending in parallel one to another and these regions comprise portions 102d, 102e can be wetted with urine or moisture contained in feces.

## Description

### TECHNICAL FIELD

The present invention relates generally to automatic urine disposal apparatuses adapted to take care of urination in an automatic fashion and thereby to assist persons for whom it is difficult to control urination timing on their own will or to clean up after urination.

### RELATED ART

Among aged and/or sick persons, there are persons for whom it is difficult to control urination timing on their own will or to clean up after urination. To assist these persons suffering from such problem, for example, JP2007-44493A discloses an automatic urine disposal apparatus. Generally, such known automatic urine handling apparatus comprises a urine suction device having a urine retainer unit put on the user's body adapted so as to cover the wearer's urethral orifice and its peripheral region and vacuum suction means such as a suction pump provided separately of the urine receiver unit so that urine collected by the urine retainer unit may be guided into a urine reservoir under action of the vacuum suction means. Air within the hermetically-sealed urine reservoir may be sucked by the suction pump to generate a differential pressure between the urine retainer unit and the urine reservoir and thereby to guide urine within the urine retainer unit into the urine reservoir.

Such urine suction device of known art further comprises a urine sensor adapted to detect urination and to generate a detection signal on the basis of which the suction pump is actuated. The urine sensor includes, in turn, a pair of electrodes arranged in parallel to and spaced from each other. When urination occurs and these two electrodes are electrically connected to each other by the intermediary of urine, a urine detecting circuit constituted by these electrodes is turned on, actuating the suction pump. With the urine suction device put on the wearer's body, a pair of electrodes extends in a vertical direction and lower ends thereof lie in the vicinity of the wearer's anus.
PATENT DOCUMENT 1: JP2007-44493A

### DISCLOSURE OF THE INVENTION

### PROBLEM TO BE SOLVED BY THE INVENTION

The known urine suction devices as has been exemplarily described are provided on the lower end thereof with a defecation sensor. For example, if loose passage moving from the anus toward the urethral orifice clings the urination sensor, the normal operation of the urination sensor will be no more expected. To avoid such situation, the urine suction device disclosed in the JP2007-44493A is constructed so that the presence of feces can be detected by the defecation sensor provided at the lower end of the detecting electrodes before loose passage reaches the urination sensor and the care personnel can be informed of this, for example, in the form of an alarm lamp blinking. However, depending on various factors such as a flow pattern of loose passage and/or whether the urine suction device is put on the wearer's body properly or improperly, the defecation sensor may not be able to properly function to detect defecation and, in consequence, the urine suction device may not be able to normally function.

It is an object of the present invention to provide an automatic urine disposal apparatus adapted to detect the presence of feces in a wide range.

### MEASURE TO SOLVE THE PROBLEM

According to the present invention, there is provided an automatic urine disposal apparatus comprising a urine suction device including a urine receiver unit adapted to be put on the wearer's body so as to face the wearer's urethral orifice and its peripheral skin and a detector unit attached to the urine receiver unit so as to be interposed between the skin and the urine receiver unit and a control unit including a vacuum suction means to which the urine suction device is detachably connected and adapted to put the interior of the urine receiver unit under a negative pressure, the detector unit having a urination detector to detect the presence of urine and a defecation detector to detect the presence of feces so that the vacuum suction means is actuated on the basis of a first detection signal from the urination detector to the urine into the urine receiver unit and the presence of the feces is detected on the basis of a second detection signal from the defecation detector.

The improvement according to the present invention is characterized as will be described below: The urination detector is adapted to output the first detection signal when the urination detector is wetted with the urine and comprises a pair of first electrodes spaced from each other and extending in one direction in parallel to each other. The defecation detector is adapted to output the second detection signal when the defecation detector is wetted with moisture contained in the feces and comprises a pair of second electrodes spaced from each other and extending the one direction in parallel to each other. The pair of first electrodes and the pair of second electrodes respectively have portions spaced from and in parallel to one another so that these portions are able to be wetted with the urine or moisture contained in the feces.

According to one preferred embodiment of the present invention, the pair of first electrodes and the pair of second electrodes are formed on one and same insulating a synthetic resin film.

According to another preferred embodiment of the present invention, the second electrodes having regions extending beyond full length of the first electrodes and the regions are wetted with moisture contained in the feces.

According to still another preferred embodiment of the present invention, the vacuum suction means is actuated when the electrical resistance between the pair of first electrodes in the urination detector decreases to a first specified resistance value or lower and the user of the automatic urine disposal apparatus is informed of the presence of the feces when the electrical resistance between the pair of second electrodes in the defecation detector remains at a second specified resistance value or lower which is set to be higher than the first specified resistance value for a predetermined time period or longer.

According to yet another preferred embodiment of the present invention, the urination detector and the control unit cooperate to actuate the vacuum suction means for a predetermined time period and then stop this when the electrical resistance between the pair of first electrodes increases from a value lower than the first specified resistance value up to a value exceeding the first specified resistance value.

According to further another preferred embodiment of the present invention, the urination detector and the control unit cooperate to stop the vacuum suction means when the electrical resistance between the pair of first electrodes remains at the specified resistance value or lower for a time period longer than a predetermined time period.

According to further alternative preferred embodiment of the present invention, a value of the second specified resistance value or lower appearing between the second electrodes is a value between the second specified resistance value and the first specified resistance value.

### EFFECT OF THE INVENTION

In the automatic urine disposal apparatus according to the present invention, the first electrodes for urine detection as well as the second electrodes for feces detection respectively have regions spaced one from another and extending in one direction in parallel one to another. With this unique design, any amount of feces being moving into the regions of the urine suction device used to detect the presence of urine can be detected by the second electrodes and the user of the automatic urine disposal apparatus such as the helper can be informed of the presence of feces.

### BRIEF DESCRIPTION OF THE DRAWINGS

[FIG. 1] Schematic diagram illustrating the automatic urine disposal apparatus including the urine suction device.
[FIG. 2] Diagram illustrating the urine suction device as put on the wearer's body.
[FIG. 3] Plan view of the urine suction device.
[FIG. 4] Sectional view taken along the line IV-IV in Fig. 3.
[FIG. 5] Sectional view taken along the line V-V in Fig. 3.
[FIG. 6] Plan view of the electrode assembly.
[FIG. 7] Sectional view taken along the line VII-VII in Fig. 6.
[FIG. 8] Sectional view taken along the line VIII-VIII in Fig. 6.
[FIG. 9] Plan view of the electrode assembly having the insulating coating peeled off.
[FIG. 10] Flow chart illustrating the steps of controlling the automatic urine disposal apparatus.

### IDENTIFICATION OF REFERENCE NUMERALS USED IN THE DRAWINGS

- 100: automatic urine disposal apparatus
- 100a: vacuum suction means
- 101: control unit
- 102: urine suction device
- 102a: urine receiver unit
- 102b: urination detector
- 102c: defecation detector
- 143a: first electrode (feces detecting electrode)
- 143b: second electrode (feces detecting electrode)
- 218a: first electrode (urine detecting electrode)
- 218b: second electrode (urine detecting electrode)

### DETAILED DESCRIPTION OF THE PREFERRED EMBODIMENTS

Details of the automatic urine disposal apparatus will be more fully understood from the description given hereunder with reference to the accompanying drawings.

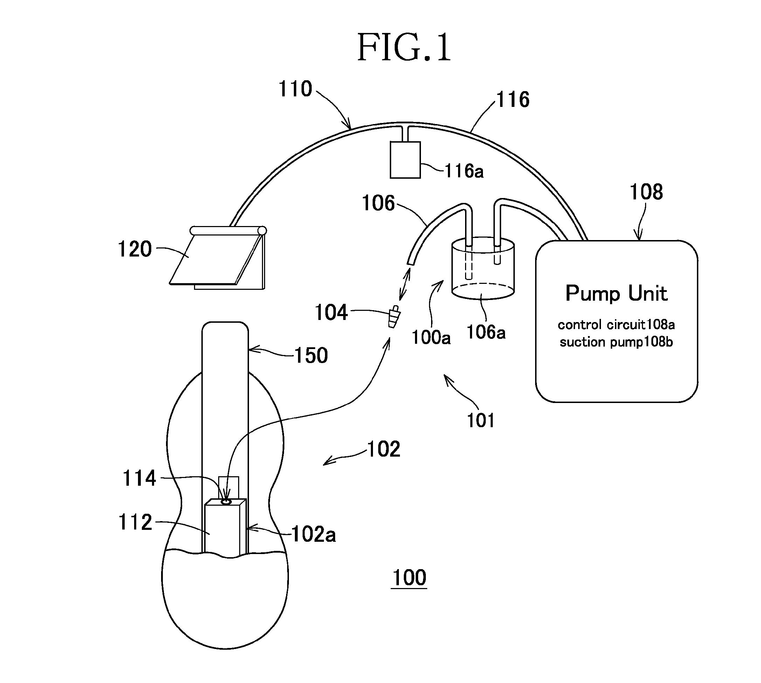

Fig. 1 is a diagram schematically illustrating an automatic urine disposal apparatus 100 comprising a urine suction device 102 according to the present invention and a control unit including a vacuum suction means 100a combined with the urine suction device 102. The urine suction device 102 has an inner side facing the wearer's skin and an outer side facing a wearer's garment. Referring to Fig. 1, the outer side is illustrated as partially cutaway.

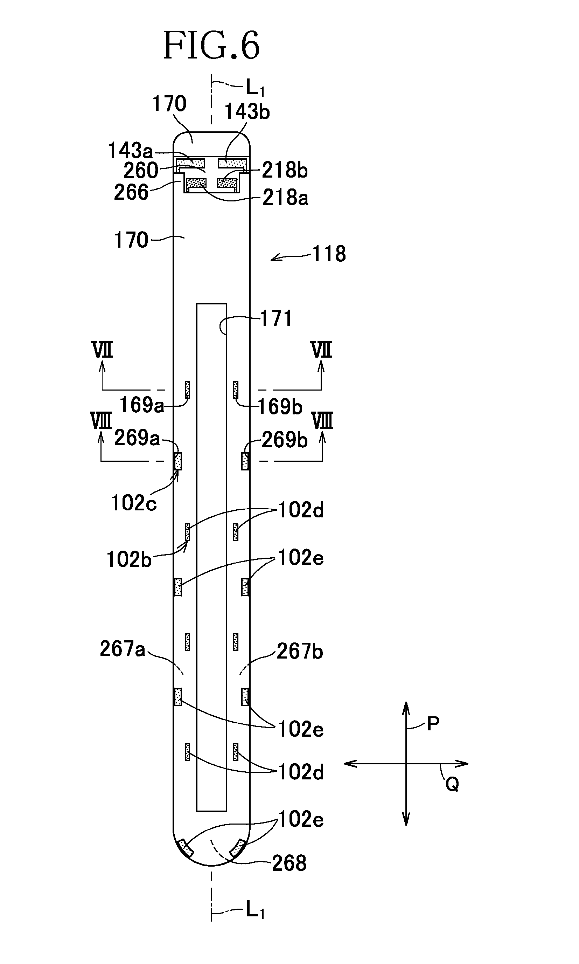

The automatic urine disposal apparatus 100 is adapted to collect urine being excreted by the wearer in the urine suction device 102 in preparation to disposal thereof. The urine suction device 102 comprises a urine receiver unit 102a and a detector unit 150. The urine receiver unit 102a is adapted to be put on the wearer's body so as to cover the wearer's urethral orifice and its peripheral region to collect urine being excreted by the wearer. The detector unit 150 comprises a urination detector 102b and a defecation detector 102c adapted to detect the presence of feces on the urination detector 102b (See Fig. 6). The vacuum suction means 100a includes a joint member 104 adapted to be connected to the urine receiver unit 102a, a urine guide tube 106, a urine reservoir 106a, a pump unit 108 and an electric wiring 116.

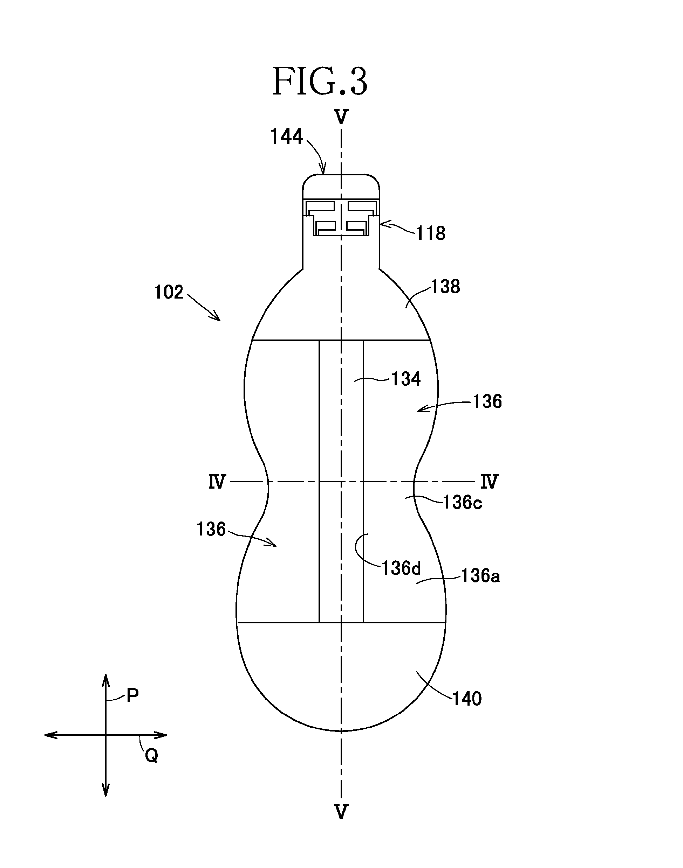

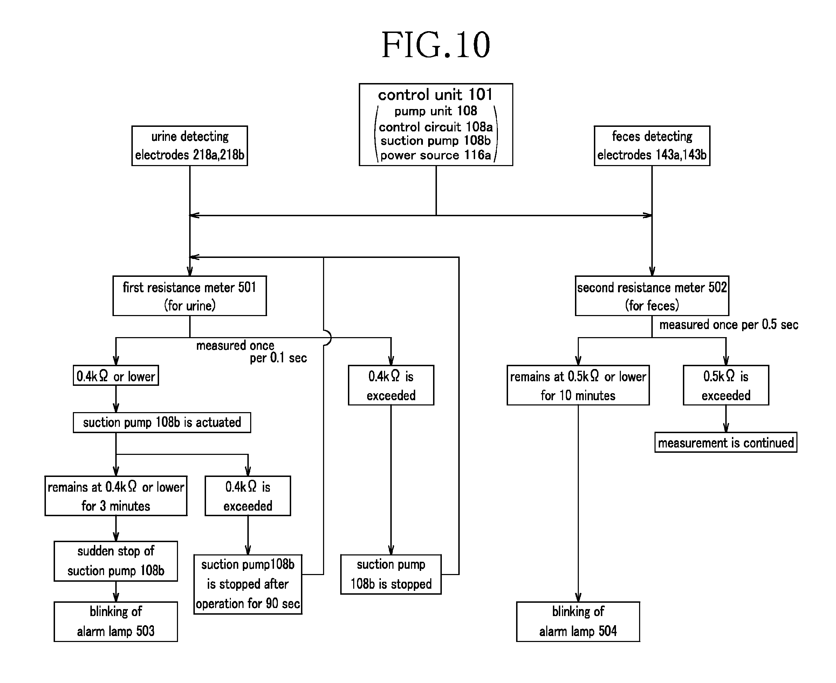

The pump unit 108 principally includes a control circuit 108a adapted to process an electric signal transmitted from the detector unit 150 via the wiring 116 and a suction pump 108b adapted to be actuated under control by the circuit 108a. In the urine suction device 102, a urine retainer 112 of the urine receiver unit 102a is provided in a peripheral wall thereof with a urine outlet 114 to which the urine guide tube 106 is connected via the joint 104. A distal end of the wiring 116 extending from the pump unit 108 is provided with a clip 120 used for electrical connection of urination detecting electrodes 218a, 218b (See Figs. 3 through 5) included in the urination detector 102b and defecation detecting electrodes 143a, 143b of the defecation detector 102c to the wiring 116. With such automatic urine disposal apparatus 100, upon urination, a detection signal is transmitted from the urination detector 102b to the pump unit 108 which actuates, in response to this signal, the suction pump 108b to suck air within the urine reservoir 106a and thereby to suck urine into the urine retainer 112. Urine sucked into the urine retainer 112 in this manner is further sucked and collected into the urine reservoir 106a via the joint 104 and the guide tube 106. If any amount of feces is detected to be present in any region of the urine suction device 102, the defecation detector 102c transmits a detection signal to the pump unit 108 whereupon the control circuit108a included in the pump unit 108 is actuated to blink an alarm lamp 504 (See Fig. 10) to inform the care personnel of the presence of feces.

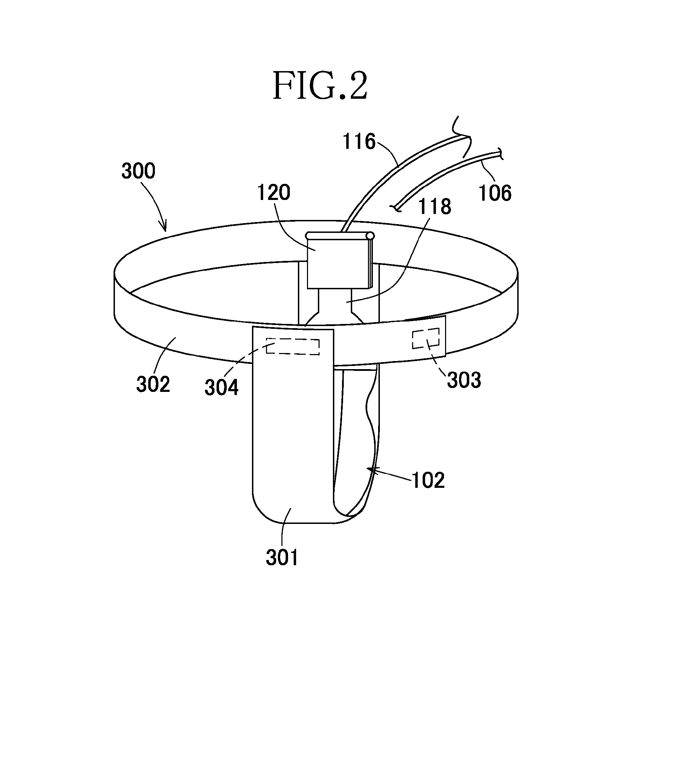

Fig. 2 is a diagram exemplarily illustrating how to put the urine suction device 102 on the wearer's body wherein the clip 120 is illustrated to lie on the ventral side. The urine suction device 102 is fixed to an inner side of a crotch belt segment 301 as a part of a T-shaped belt 300, for example, by pressure-sensitive adhesive or a mechanical fastener known by the trade name "Velcro". The urine suction device 102 is put on the wearer's body so that the urine retainer 112 extends for the most part thereof in a longitudinal direction on the wearer's ventral side with its inner side opposed to the wearer's urethral orifice and a peripheral region thereof and with its lower end extending toward the anus along an inner surface of the crotch belt segment 301 so as to describe a gentle curve. In the T-shaped belt 300, opposite ends of a waist belt segment 302 are detachably connected to each other by means of connecting means 303 such as a mechanical fastener while the crotch belt segment 301 is sutured at one end to the waist belt segment 302 and detachably connected at the other end to the waist belt segment 302 by means of a mechanical fastener 304. The chassis for the urine suction device 102 is not limited to the T-shaped belt 300 and the other appropriate means such as open-type diapers, pants-type diapers, diaper covers and incontinent pants may be used as the chassis of this urine suction device 102.

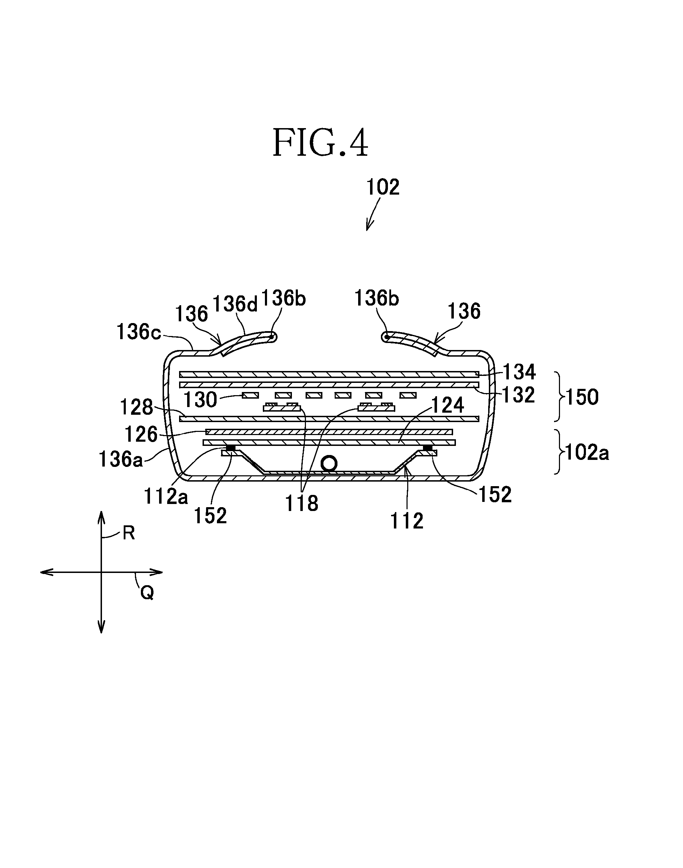

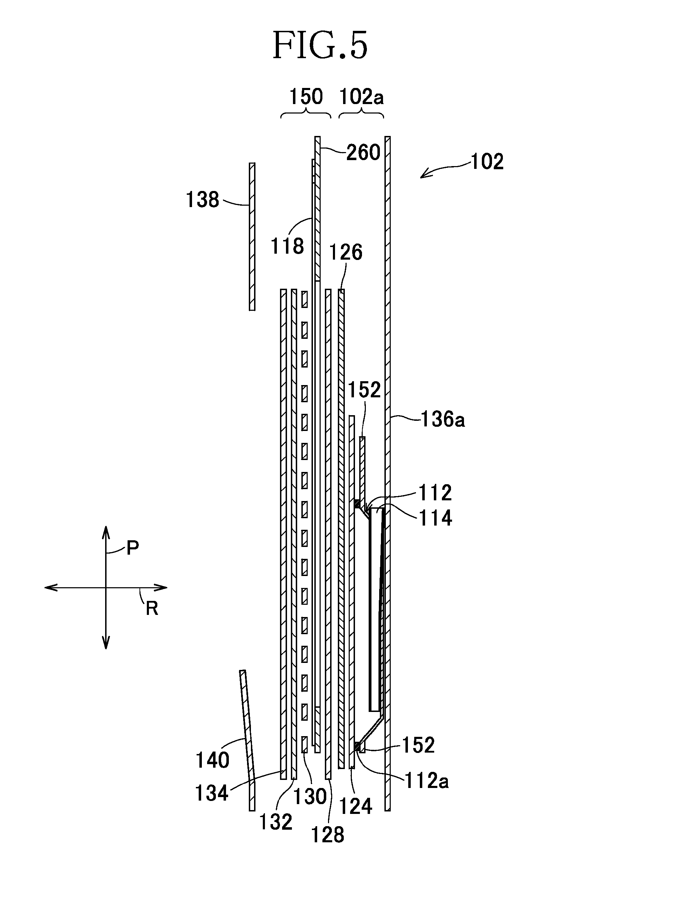

Fig. 3 is a plan view showing the inner side of the urine suction device 102, Fig. 4 is a sectional view taken along the line IV-IV in Fig. 3, and Fig. 5 is a sectional view taken along the line V-V in Fig. 3. In Figs. 4 and 5, respective members overlapping one another in a thickness direction R of the urine suction device 102 are substantially illustrated to be spaced one from another. The thickness direction R corresponds to a depth direction of the urine receiver unit 102a.

The urine suction device 102 has a longitudinal direction P corresponding to the same direction of the wearer's body and a transverse direction Q extending orthogonally to the longitudinal direction P. The urine suction device 102 has a relatively large width in the vicinity of ends opposite in the longitudinal direction P and a relatively small width in a middle as viewed in the direction P. The urine suction device 102 has also the thickness direction R and as apparent from Fig. 4, comprises a plurality of sheet members overlapping one another, i.e., a liquid-pervious but air-permeation retardant sheet 124, a liquid-dispersible sheet 126, a cushion sheet 128, the electrode assembly 118, a spacer 130, a filter 132 and a liquid-pervious skin-contact sheet 134 in this order from the bottom as viewed in the thickness direction R. A pair of leak-barriers 136 overlaps the liquid-pervious skin-contact sheet 134. The air-permeation retardant sheet 124 and the liquid- dispersible sheet 126 are integrated with the urine retainer 112 to form the urine receiver unit 102a. The cushion sheet 128, the electrode assembly 118, the spacer 130, the filter 132 and the skin-contact sheet 134 overlap one another to form the detector 150.

The urine retainer 112 is provided in the form of a tray and made of a soft elastic material such as soft polyethylene or silicon rubber so as to be flexible in the longitudinal direction P as well as in the transverse direction Q but well resistant to any deformation due to a negative pressure exerted thereon during urine suction by the suction pump. The air-permeability retardant sheet 124 is bonded to a peripheral flange 152 of the urine retainer 112 along a joint region 112a. The depth direction of the urine retainer 112 corresponds to the thickness direction R.

The air-permeation retardant sheet 124 is significantly liquid-pervious but substantially or completely air-impermeable. As will be apparent from Fig. 4, this sheet 124 covers an upper opening of the urine retainer 112. The urine retainer 112 provided with the air-permeation retardant sheet 124 in this manner is readily put under a negative pressure as soon as the suction pump 108b of the pump unit 108 is actuated and urine collected in the urine retainer 112 can be quickly sucked by the suction pump 108b. As stock materials for the air-permeation retardant sheet 124, for example, an SMS nonwoven fabric consisting of a spun bond nonwoven fabric having a basis weight of 22g/m², a melt blown nonwoven fabric having a basis weight of 10g/m² and a spun bond nonwoven fabric having a basis weight of 22g/m², preferably modified by surfactant to become hydrophilic. On the basis of the result obtained from measurement of air-permeability carried out according to Method A selected from Method for Air-permeability Measuring Methods prescribed in Section 6. 27. 1 of JIS L 1096 is, in wet condition, air-permeability of the air-permeation retardant sheet 124 is in a range from 0 to 100 cc/cm²/sec., preferably in a range from 0 to 50 cc/cm²/sec. In a dry condition, this value is in a range from 20 to 200 cc/cm²/sec., preferably in a range from 20 to 100 cc/cm²/sec., more preferably in a range from 20 to 50 cc/cm²/sec. The term "wet condition" used herein for measurement of the air-permeability refers to a condition in which a moisture content of the air-permeation retardant sheet 124 calculated by a following formula (1) is 100% or higher while the term "dry condition" refers to the condition of this air-permeation retardant sheet 124 after this sheet has been left in a room at a temperature of 20°C and an RH of 50%. Moisture content = (weight of sheet in a wet condition - weight of sheet in a dry condition) / (weight of sheet in dry condition) Formula (1)

The liquid-dispersible sheet 126 is formed of a liquid-pervious sheet such as a nonwoven fabric containing hydrophilic fibers such as rayon fibers and serves to disperse urine rapidly over the air-permeation retardant sheet 124 upon urination and thereby to make the air-permeation retardant sheet 124 over an area as large as possible in a wet condition. The air-permeation retardant sheet 124 in such wet condition ensures that a negative pressure is generated within the urine retainer 112 and, in consequence, urine suction into the urine retainer 112 is facilitated. Preferably, the liquid-dispersible sheet 126 is intermittently bonded to the air-permeation retardant sheet 124 to avoid a problem that the liquid-pervious properties of these two sheets might be deteriorated.

The cushion sheet 128 is formed of a liquid-pervious sheet such as a thermal bond nonwoven fabric having a basis weight of 20 to 30 g/m² adapted to promote permeation of urine and thereby to prevent any amount of urine present in the liquid-dispersible sheet 126 and the air-permeation retardant sheet 124 from flowing back toward the electrode assembly 118. Furthermore, the sheet-like members such as the electrode assembly 118, the spacer 130 and the filter 132 may be previously overlapped upon the cushion sheet 128 to make it possible for the cushion sheet 128 to serve as a carrier member used to hold these sheet-like members at predetermined positions in the urine suction device 102 in the course of manufacturing the urine suction device 102. Preferably, the cushion sheet 128 is intermittently bonded to the liquid-dispersible sheet 126 in order not to deteriorate the liquid-pervious property of these two sheets 128, 126.

The electrode assembly 118 is obtained, for example, by printing the electrode pair of desired shape on a synthetic resin film with conductive ink and structural details thereof will be described later. The electrode assembly 118 may be bonded to the cushion sheet 128.

The spacer 130 is thicker than any other sheet-like members in the detector unit 150 and provided in the form of a mesh-textured liquid-pervious sheet. In the urine suction device 102, even after operation of suction, the skin-contact sheet 134 might remain in wet condition due to any amount of urine still staying thereon. In this case, the skin-contact sheet 134 might come in direct or indirect contact with the electrode assembly 118, for example, under the wearer's body weight and cause a false operation of the electrode assembly 118. The spacer 130 functions as means to keep the electrode assembly 118 and the filter 132 spaced from each other and thereby to prevent the false operation of the electrode assembly 118. More specifically, the spacer 130 is not responsible for urine suction but water repellent and has an air-permeability as well as a liquid-permeability higher than those of the air permeation retardant sheet 124. The spacer 130 maintains its thickness constant even under the wearer's body weight. Such spacer 130 can be formed by a mesh textured sheet having a thickness of 0.5 to 1 mm made of a soft synthetic resin such as vinyl acetate and is preferably bonded to the cushion sheet 128 in a manner that the liquid-permeability of these sheet materials might not be adversely affected.

The filter 132 is used to prevent any solid material contained in urine from accumulating on the electrode assembly 118 and becoming permanently conductive and, in view of this, the filter 132 is preferably formed by the sheet having air-permeability and liquid-permeability both higher than those of the air-permeation retardant sheet 124 and more preferably formed of a nonwoven fabric. The filter 132 may be bonded to spacer 130 in a manner that the liquid-permeability of these sheet materials might not be adversely affected.

The skin-contact sheet 134 overlies the filter 132 and is adapted to face and come in contact with the wearer's urethral orifice and peripheral region thereof when the urine suction device 102 is put on the wearer's body. Such skin-contact sheet 134 may be formed of a soft and liquid-pervious sheet material such as a thermal bond nonwoven fabric having a basis weight of 15 to 25 g/m². Similarly to the cushion sheet 128, the skin-contact sheet 134 is instantaneously impregnated with urine at an initial stage of urination. The skin-contact sheet 134 is bonded to the filter 132 preferably in the intermittent fashion in order to prevent the liquid-permeability of these sheets 134, 132 from being adversely affected by this bonding treatment. The skin-contact sheet 134 may be hydrophilic or water-repellent.

The leak-barrier 136 is paired in right and left barriers as will be seen in Figs. 3 and 4 and adapted to prevent any amount of urine might flow on the skin-contact sheet 134 in the transverse direction Q and leak sideways out from the urine suction device 102. The leak-barrier 136 shown in Fig.4 has its outer side edge 136c lying aside toward the outer side edge of the urine suction device 102 is bonded to the skin-contact sheet 134 while its inner side edge 136d lying aside toward the middle zone of the urine suction device 102 is not bonded to the skin-contact sheet 134 and provided with an elastic member 136b such as rubber thread bonded under tension thereto so as to extending in the longitudinal direction P. A sheet 136a which is forming a pair of leak-barriers 136 covers the bottom of the urine retainer 112. With the urine suction device 102 being put on the wearer's body, it bows in the longitudinal direction P as will be seen in Fig. 1 under contraction of the elastic member 136b and thereupon the side edge 136d of the leak-barrier 136 is spaced upward from the skin-contact sheet 134. A sheet 136a constituting the leak-barrier 136 is preferably liquid-impervious and may be formed, for example, by a soft thermoplastic synthetic resin film or a composite sheet consisting of such film and a nonwoven fabric. As viewed with the urine suction device 102 being flattened (See Fig. 3), upper and lower ends of the leak-barrier 136 are covered with first and second end sheets 138, 140, respectively.

Fig. 6 is a plan view of the electrode assembly 118 used in the device illustrated by Figs. 3, 4 and 5. The electrode assembly 118 comprises insulating film member 260 formed of a synthetic resin film, a pair of urine detecting electrodes 218a, 218b and a pair of feces detecting electrodes 143a, 143b formed on one and the same surface of the film member 260 and insulating coating 170 with which the most part of these electrodes 218a, 218b, 143a, 143b are coated. The film member 260 is a rectangular member extending in the longitudinal direction P and a middle zone of this rectangular film member 260 as viewed in the transverse direction Q is cut out in the longitudinal direction P so as to form a substantially rectangular opening 171. Such film member 260 has an upper end 266 adapted to hold the clip 120 as seen in an upper portion of Fig. 6, lateral zones 267a, 267b extending downward from the upper end 266 on both sides of a center line L₁-L₁ bisecting a width of the electrode assembly 118, and lower ends 268 extending downward from the lateral zones 267a, 267b, respectively. At the upper end 266 of the film member 260, the electrodes 218a, 218b as well as the electrodes 143a, 143b are exposed. In the lateral zones 267a, 267b, the insulating coating 170 is formed with a plurality of relatively narrow spots as first non-coated regions 169a, 169b to define exposed regions 102d free from the insulating coating 170, allowing the urine detecting electrodes 218a, 218b to be wetted with urine. In the lateral zones 267a, 267b, the insulating coating 170 is further formed with a plurality of relatively wide spots as second non-coated regions 269a, 269b to define exposed regions 102e free from the insulating coating 170, allowing the feces detecting electrodes 143a, 143b to be wetted with moisture contained in feces. The exposed regions 102e in the lower end 268 is to lie in the vicinity of the anus when the urine suction device 102 is put on the wearer's body and effective for rapid detection of feces being entering the urine suction device 102.

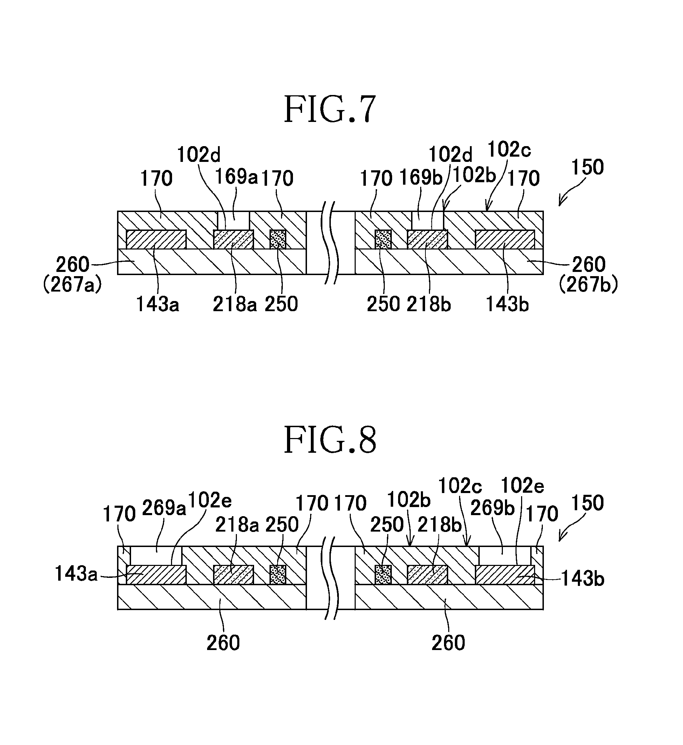

Fig. 7 is a sectional view taken along the line VII-VII in Fig. 6, showing the exposed regions 102d of the urine detecting electrodes 218a, 218b. Referring to Fig. 7, a circuit 250 and the feces detecting electrodes 143a, 143b are covered with the insulating coating 170.

Fig. 8 is a sectional view taken along the line VIII-VIII in Fig. 6, showing a manner in which the feces detecting electrodes 143a, 143b are exposed in the regions 102e. Referring to Fig. 8, the circuit 250 and the urine detecting electrodes 218a, 218b are covered with the insulating coating 170.

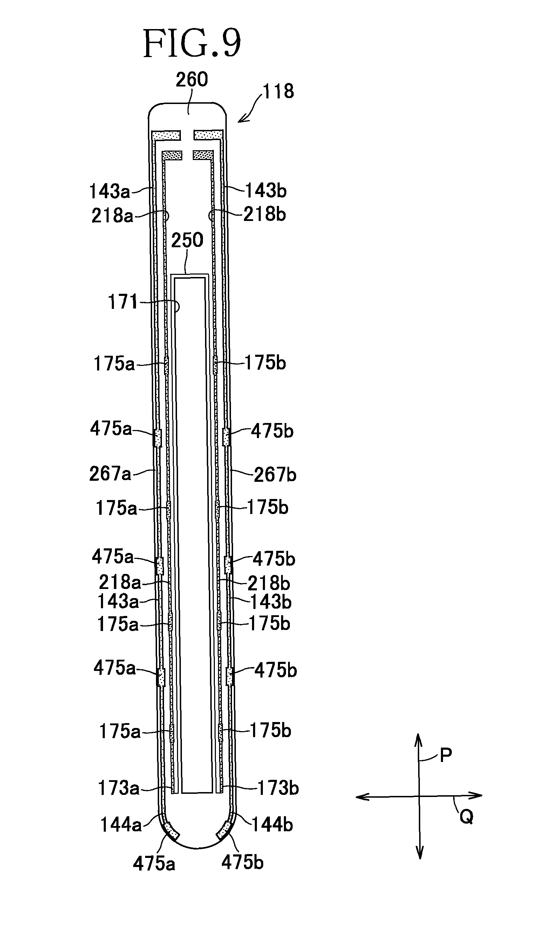

Fig. 9 is a plan view showing the electrode assembly 118 having the insulating coating 170 peeled off. The film member 260 is provided in the lateral zones 267a, 267b thereof with a pair of the urine detecting electrodes 218a, 218b extending in the longitudinal direction P so as to be parallel to and spaced from each other. A plurality of portions 175a, 175b distributed in the respective electrodes 218a, 218b are exposed in the first non-coated regions 169a, 169b as shown in Fig. 6. Between the urine detecting electrodes 218a, 218b, the breaking detector circuit 250 is formed. The breaking detector circuit 250 is electrically connected with the respective lower ends 173a, 173b of the respective urine detecting electrodes 218a, 218b and extends along the peripheral edge of the opening 171 as illustrated. The film member 260 is further provided in the lateral zones 267a, 267b thereof with a pair of the feces detecting electrodes 143a, 143b extending in the longitudinal direction P so as to be parallel to and spaced from each other. A plurality of portions 475a, 475b of these feces detecting electrodes 143a, 143b are exposed in the second non-coated regions 269a, 269b as shown by Fig. 6. Lower ends 144a, 144b of the respective feces detecting electrodes 143a, 143b extend downward beyond the lower ends of the respective urine detecting electrodes 218a, 218b and the lower ends 144a, 144b also are formed with the portions 475a, 475b.

In the electrode assembly 118, the film member 260 is preferably formed by polyester film having a thickness of 50 to 100 µm. The urine detecting electrodes 218a, 218b may be obtained by printing them in desired shapes on the film member 260 with conductive ink or conductive coating material. The conductive ink or the conductive coating material may contain, for example, carbon black of 3 to 7% by weight, artificial graphite such as carbon graphite of 10 to 30% by weight and an appropriate quantity of silver pigment. Each of the urine detecting electrodes 218a, 218b is configured to have a width of 0.5 to 2.0 mm and a resistance value of 150 KΩ or lower wherein each of the portions 175a, 175b may have an appropriate width to be easily exposed in the first non-coated regions 169a, 169b. The breaking detector circuit 250 may be obtained, for example, by printing them in desired shape on the film member 260 with ink containing carbon black in 3 to 7% by weight and artificial graphite in 5 to 10% by weight. It is essential for this circuit 250 to exhibit a resistance value substantially higher than a resistance value exhibited by the urine detecting electrodes 218a, 218b and preferably has a width of 0.3 to 1 mm and a resistance value of 2 to 10 MΩ. The feces detecting electrodes 143a, 143b also may be obtained by printing them on the film member 260 with the same ink or coating material as those used for the urine detecting electrodes 218a, 218b and sometimes by vacuum deposition of aluminum. Each of the feces detecting electrodes 143a, 143b also is configured to have a width of 0.5 to 2.0 mm wherein the portions 475a, 475b may have an appropriate width to be easily exposed in the second non-coated regions 269a, 269b. An electric resistance between the feces detecting electrodes 143a, 143b which are spaced from and parallel to each other is infinite.

When the electrode assembly 118 and the control unit 101 are electrically connected with each other via the clip 120, the urine detecting electrodes 218a, 218b as well as the feces detecting electrodes 143a, 143b are supplied with weak current from a power source 116a (See Fig. 1). In the control circuit 108a for the pump unit 108, an electrical resistance between the urine detecting electrodes 218a, 218b or the other physical value corresponding to this electrical resistance as is continuously or intermittently measured along with an electrical resistance between the feces detecting electrodes 143a, 143b or the other physical value corresponding to this electrical resistance. It should be appreciated here that the urine detecting electrodes 218a, 218b are electrically connected with each other via the breaking detector circuit 250 and a weak current passing them is detected by the control circuit 108a. When such weak current can not be detected even after a predetermined time period has elapsed, it will be determined that the urine detecting electrodes 218a, 218b are out of order and the control circuit 108a generates an alarm to the user of the automatic urine disposal apparatus 100. Occurrence of urination in the urine suction device 102 causes the exposed portions 102d of the urine detecting electrodes 218a, 218b to be electrically connected to each other to reduce an electrical resistance between the urine detecting electrodes 218a, 218b and, on the basis of such reduced resistance represented in the form of a detection signal, the control circuit 108a determines that urine is present in the urination detector 102b, i.e., urination has occurred and actuates the suction pump 108b. A degree of such resistance reduction depends on various conditions of the urine suction device 102 such as areas at which the urine detecting electrodes 218a, 218b are exposed in the first non-coated regions 169a, 169b. In view of this, the illustrated embodiment of urine suction device 102 is designed so that the electrical resistance between the urine detecting electrodes 218a, 218b is reliably reduced down to 0.4 kΩ or lower in response to urination and this electrical resistance of 0.4 kΩ or lower lasting for a predetermined time period, e.g., 0.2 sec. is set as a critical resistance value, i.e., a threshold value to actuate the suction pump 108b. The suction pump 108b is preferably able to complete urine suction by the urine suction device 102 within 1 to 2 minutes and, so far as the suction pump having such ability is used, it can be determined that the automatic urine disposal apparatus 100 is out of order if operation of the suction pump 108 continues, for example, for 3 minutes or longer.

If loose passage moves into the urine suction device 102 put on the wearer's body and the exposed portions 102e of the paired feces detecting electrode 143a, 143b are electrically connected with each other via moisture contained in such loose passage, the electrical resistance between these feces detecting electrodes 143a, 143b is reduced. Generally, such reduction of the electrical resistance due to the presence of feces is less remarkable than reduction of the electrical resistance due to the presence of urine. However, reduction of the electrical resistance due to the presence of feces also depends on various conditions of the urine suction device 102 and, in view of this, the illustrated embodiment of the pump unit 108 is designed so that the electrical resistance between the feces detecting electrodes 143a, 143b is reliably reduced down to 0.5 kΩ or lower in response to defecation and this electrical resistance higher than 0.4 kΩ lasting for a predetermined time period, e.g., 10 minutes is set as a critical resistance value, i.e., a threshold value on the basis of which an alarm is generated to remind exchange of the urine suction device 102.

In the detector unit 150, a pair of the urine detecting electrodes 218a, 218b, the film member 260 and the insulating coating material 170 cooperated together to form the urination detector 102b while a pair of feces detecting electrodes 143a, 143b, the film member 260 and the insulating coating material 170 cooperate together to form the defecation detector 102c. The urine detecting electrodes 218a, 218b as well as the feces detecting electrodes 143a, 143b are respectively spaced from each other and extend in parallel in the longitudinal direction P as will be seen in Fig. 9. These electrodes respectively have the exposed portions 102d, 102e formed intermittently in the longitudinal direction P and all of these electrodes are formed on one and same surface of the single film member 260. With this unique construction, the automatic urine disposal apparatus 100 can quickly detect the presence of feces in the region of the detector unit 150 allocated for detection of urine and thereby can effectively prevent the automatic urine disposal apparatus 100 from being put in abnormal situation in which the apparatus otherwise could not achieve the desired urine suction due to the presence of feces.

A pair of the urine detecting electrodes 218a, 218b and a pair of the feces detecting electrodes 143a, 143b are formed on one and same surface of the film member 260 and a distance between these electrodes as viewed in the depth direction of the urine retainer 112 corresponding to the thickness direction R in Fig. 4 is 0 mm so far as the illustrated embodiment is concerned. However, the electrode assembly 118 available for the present invention is not limited to such illustrated embodiment. For example, it is possible to print a pair of the urine detecting electrodes 218a, 218b and a pair of the feces detecting electrodes 143a, 143b on two separate film members so as to obtain the electrode assembly 118. It is also possible to sandwich nonwoven fabric between these two film members printed with the electrodes, then to bond these two film members to the nonwoven fabric and thereby to obtain the electrode assembly 118. In any case, it should be noted that a pair of urine detecting electrodes 218a, 218b and a pair of the feces detecting electrodes 143a, 143b are arranged close to one another as viewed in the depth direction of the urine retainer 112 preferably so as to be spaced from one another only by a distance of 0 to 2 mm in order to ensure that urine as well as feces can be detected as soon as urination as well as defecation occurs.

In the control unit 101, the urine detecting electrodes 218a, 218b are adapted to detect a specified resistance value of 0.4 kΩ and the feces detecting electrodes 143a, 143b are adapted to detect a specified resistance value higher than 0.4 kΩ. Consequentially, a malfunction would not occur in the automatic urine disposal apparatus 100 even when the feces detecting electrodes 143a, 143b are electrically connected with each other via the presence of urine and the urine detecting electrodes 218a, 218b are electrically connected with each other via the presence of feces.

Fig. 10 is a control flow chart for the automatic urine disposal apparatus 100 with the urine suction device 102 electrically connected to the control unit 101 shown in Fig. 1. The control unit 101 has a power source 116a (See Fig. 1) adapted to supply the urine suction device 102 with weak current in continuous manner and the control circuit 108a measures electrical resistance or the other physical quantity such as voltage appearing between the urine detecting electrodes 218a, 218b and electrical resistance or the other physical quantity such as voltage appearing between the feces detecting electrodes 143a, 143b. On the basis of variation appearing, for example, in the electrical resistance, the control circuit 108a actuates or stops the suction pump 108b and informs the care personnel of the abnormal situation by blinking the alarm lamps 503, 504. The control flow chart of Fig. 10 exemplarily indicates a particular embodiment of operating conditions of the automatic urine disposal apparatus 100. According to this particular embodiment, the urine detecting electrodes 218a, 218b and the feces detecting electrodes 143a, 143b are supplied from the power source 116a with current. In the pump unit 108, electrical resistance between the urine detecting electrodes 218a, 218b is measured by a first resistance meter 501 once per 0.1 sec. If the electrical resistance exceeds the specified resistance value of 0.4 kΩ, it is determined that no urination occurs and the suction pump 108b is left stopped. If the electrical resistance drops to a level lower than 0.4 kΩ at least for 0.2 sec, it is determined that urination has occurred and the suction pump 108b is actuated so that the urine suction device 102 begins to suck urine. When the electrical resistance rises again from the level lower than 0.4 kΩ up to 0.4 kΩ or higher, it is determined that urine suction has been completed and the suction pump 108b is actuated for further 90 sec to dry the skin-contact sheet 134 of the urine suction device 102 and then stopped. If the electrical resistance remains lower than 0.4 kΩ for 3 minutes, it is determined that the urine suction device 102 is out of order and the suction pump 108b is stopped. Thereupon, the alarm lamp 503 is blinked to remind the care personnel to check the urine suction device 102.

In the pump unit 108, electrical resistance between the feces detecting electrodes 143a, 143b is measured by a second resistance meter 502 once per 0.5 sec. If the electrical resistance is measured to exceed the specified resistance value of 0.5 kΩ, it is determined that no feces is present in the urine suction device 102 and the resistance measurement is continued. If the electrical resistance decreases to a level lower than 0.5 kΩ and remains at such lower level for 10 minutes, it is determined that any amount of feces being moving into the urine suction device 102. On the basis of such determination, the alarm lamp is blinked to remind the care personnel to exchange the urine suction device 102. It is essential to set the electrical resistance as the threshold value for blinking of the alarm lamp 504 to be higher than the specified resistance value as the threshold value for actuation of the suction pump 108b.

## Claims

1. An automatic urine disposal apparatus comprising a urine suction device including a urine receiver unit adapted to be put on the wearer's body so as to face the wearer's urethral orifice and its peripheral skin and a detector unit attached to said urine receiver unit so as to be interposed between said skin and said urine receiver unit and a control unit including a vacuum suction means to which said urine suction device is detachably connected and adapted to put the interior of said urine receiver unit under a negative pressure, said detector unit having a urination detector to detect the presence of urine and a defecation detector to detect the presence of feces so that said vacuum suction means is able to be actuated on the basis of a first detection signal from said urination detector to suck said urine into said urine receiver unit and the presence of said feces is detected on the basis of a second detection signal from said defecation detector, said automatic urine disposal apparatus further comprises:
said urination detector being adapted to output said first detection signal when said urination detector is wetted with said urine and comprises a pair of first electrodes spaced from each other and extending in one direction in parallel to each other;
said defecation detector being adapted to output said second detection signal when said defecation detector is wetted with moisture contained in said feces and comprises a pair of second electrodes spaced from each other and extending said one direction in parallel to each other; and
said pair of first electrodes and said pair of second electrodes respectively having portions spaced from and in parallel to one another so that these portions are able to be wetted with said urine or moisture contained in said feces.

2. The automatic urine disposal apparatus as defined by Claim 1, wherein said pair of first electrodes and said pair of second electrodes are formed on one and the same insulating synthetic resin film.

3. The automatic urine disposal apparatus as defined by Claim 1 or 2, wherein said second electrodes having regions extending beyond full length of said first electrodes and said regions are wetted with moisture contained in said feces.

4. The automatic urine disposal apparatus as defined by any one of Claims 1 through 3, wherein said vacuum suction means is actuated when an electrical resistance between said pair of first electrodes in said urination detector decreases to a first specified resistance value or lower and said user of said automatic urine disposal apparatus is informed of the presence of said feces when an electrical resistance between said pair of second electrodes in said defecation detector remains at a second specified resistance value or lower which is set to be higher than said first specified resistance value for a predetermined time period or longer.

5. The automatic urine disposal apparatus as defined by Claim 4, wherein said urination detector and said control unit cooperate to actuate said vacuum suction means for a predetermined time period and then stop said vacuum suction means when said electrical resistance between said pair of first electrodes increases from a value lower than said first specified resistance value up to a value exceeding said first specified resistance value.

6. The automatic urine disposal apparatus as defined by Claim 4 or 5, wherein said urination detector and said control unit cooperate to stop said vacuum suction means when said electrical resistance between said pair of first electrodes remains at said specified resistance value or lower for a time period longer than a predetermined time period.

7. The automatic urine disposal apparatus as defined by any one of Claims 4 through 6, wherein a value of said second specified resistance value or lower appearing between said second electrodes is a value between said second specified resistance value and said first specified resistance value.
